Europäisches Patentamt

European Patent Office

Office européen des brevets

(19)

(11) Publication number: **0 104 933**
A2

# (12) EUROPEAN PATENT APPLICATION

(21) Application number: **83305758.1**

(22) Date of filing: **27.09.83**

(51) Int. Cl.³: **A 61 K 37/36**
//A23K1/165

(30) Priority: **28.09.82 US 425901**

(43) Date of publication of application:
**04.04.84 Bulletin 84/14**

(84) Designated Contracting States:
**AT BE CH DE FR GB IT LI LU NL SE**

(71) Applicant: SDS BIOTECH CORPORATION
P.O. Box 348 7528 Auburn Road
Painesville Ohio 44077(US)

(72) Inventor: Duane, Warren C.
7950 Garfield Road
Mentor Ohio 44060(US)

(72) Inventor: Stutz, Max W.
57 New Castle Lane
Mentor Ohio 44060(US)

(74) Representative: Oliver, Roy Edward
POLLAK MERCER & TENCH High Holborn House 52-54
High Holborn
London WC1V 6RY(GB)

(54) **Somatomedin growth promoter.**

(57) A significant growth promoting effect and improvement in feed efficiency has been demonstrated by the administration of somatomedin to pituitary normal economic animals, e.g., sheep, cattle, chickens and swine. The growth promoting effect and improvement in feed efficiency for these animals is particularly pronounced when the administration of the somatomedin is effected in a continuous manner.

EP 0 104 933 A2

SOMATOMEDIN GROWTH PROMOTER

BACKGROUND OF THE INVENTION

(1) Field of the Invention

This invention relates generally to the use of somatomedin to promote the growth and improve the feed efficiency of economic animals. This invention also relates to compositions comprising somatomedin useful for administration to economic animals.

More specifically, the invention relates to the continuous administration of somatomedin to pituitary normal economic animals to promote the growth and improve the feed efficiency of these animals.

(2) State of the Art

It was first hypothesized by Salmon and Daughaday over 20 years ago that stimulation of growth by growth hormone (somatotropin) was mediated by a circulating factor which they called "sulfation factor." From this initial hypothesis has evolved an entire field of research directed to the characterization of the factor and how it affects the promotion of growth in an animal or human. Because of experimental evidence that circulating "sulfation factors" had the potential to promote growth and anabolism of different tissues, the term "sulfation factor" was replaced by "somatomedin." The current status of the research relating to somatomedins is reviewed in an article by Daughaday in Proc. Soc. for Exper'l. Biol. and Med., 170 (1982), pages 257-263.

Somatomedins are polypeptide hormones which are generated in the liver and possibly in other organs, in response to the release of somatotropin from the brain's pituitary gland. The knowledge concerning the in vivo action relationship of somatotropin and somatomedin is incomplete. Exogenous somatomedin has been administered only to hypopituitary, dwarf animals, or to surgically prepared hypopituitary (hypophysectomized) animals. In such cases, somatomedin has been shown to be mildly effective in stimulating growth. Data regarding the improvement of feed efficiency in these animals has not been reported. The recently reported study by Schoenle et al in Nature, Vol. 296, 18 March 1982, pages 252-253, is representative of such studies.

Furthermore, a growth hormone fraction, specifically a bovine growth hormone fraction, characterized by a molecular weight of 5000 daltons, a chain length of about 38 amino acids, and a very specific and determinable amino acid analysis, has been found useful for the acceleration of somatic growth in humans. The use of the polypeptide fraction and its separation from bovine growth hormone is described in U.S. Patents 3,664,925; 3,904,753; and 4,056,520 to Sonenberg and Yamasaki. It is also disclosed in these patents that the active fraction would appear to have utility in nonhuman applications, e.g., accelerating animal growth.

## SUMMARY OF THE INVENTION

It has been discovered, in accordance with the present invention, that the administration of exogeneous somatomedin to pituitary normal animals results in the promotion of growth in the animals and improved feed efficiency.

Further in accordance with the invention, methods for the continuous delivery of somatomedin to pituitary normal animals in preselected nontoxic dosage unit forms or preparations to promote growth and feed efficiency in the animals are provided.

Still further in accordance with the present invention, stable, nontoxic preparations of somatomedins are contemplated which are adaptable for continuous administration, e.g., subcutaneous, intravenous, etc., in a slow release formulation to pituitary normal animals to promote their growth and improve feed efficiency.

These and similar aspects, advantages and features will be appreciated by those skilled in the art upon the reading and understanding of the specification.

## DESCRIPTION OF THE PREFERRED EMBODIMENTS

It has been discovered that exogeneous somatomedin administered in a continuous manner in a slow release formulation to pituitary normal animals can effectively promote their growth and improve feed efficiency. In the past, this effect has only been demonstrated to a limited extent in hypopituitary dwarf animals or surgically modified, e.g., hypophysectomized, animals.

As used throughout the instant specification and claims, the expression "pituitary normal" animals are animals which have normal pituitary function, i.e., no congenital hypopituitary condition. Furthermore, such animals are without any endocrine surgical modifications, i.e., hypophysectomy, orchiectomy, adrenalectomy, or any similar alteration or modification. The expression "economic animals" as used herein is intended to include all animals raised for food products or other marketable products. Such animals include sheep, hogs, cattle, chickens, rabbits and the like.

The source of somatomedin for the purposes of this invention is bovine serum. However, it should be recognized by those skilled in the art that somatomedin may be obtained from several different sources, e.g., bovine cartilage, human plasma, rat liver and the like. Bovine serum was selected as a source of somatomedin for the purposes of this invention because of its availability and because beef animals were considered the appropriate target for treatment with this material. Methods for the separation and isolation of somatomedins have been described in the art. For example, such methods are described by Van Wyk et al in Annual Rev. of Med., Vol. 26 (1975), pages 427-441, W. P. Crege, Editor, and by Bala et al, Can. J. Biochem., Vol. 57 (1979), pages 1289-1298. One technique for the isolation and purification of somatomedin from bovine serum is described by Liberti in Biochem. and Biophys. Res. Commns., Vol. 67, No. 3 (1975), pages 1226-1233. However, any of the aforementioned or other techniques described in the art may be employed to effect the separation of somatomedin from its source materials.

The active somatomedin separated from bovine serum, in accordance with the present invention, is found to be a single polypeptide having a molecular weight of about 8000 daltons as determined by sephadex column chromatography and an isoeletric point of about 8.5. The somatomedin of the present invention was also found to stimulate sulfate uptake as determined by the method of Hall, _Acta Endocrinol_, 63: 338 (1970), as modified by Jennings, _Acta Endocrinol_, 94: 480 (1980).

The activity, i.e., the ability to promote growth and improve feed efficiency, of the bovine somatomedin of the present invention has been demonstrated through testing somatomedin in pituitary normal, young male mice and rats. The bovine somatomedin was administered to the pituitary normal rats by continuous methods both through subcutaneous and intravenous routes. Both routes were found to improve growth and feed efficiency. A preferred embodiment of the present invention for the continuous administration of bovine somatomedin to an animal is by an implanted Alzet Minipump$^{TM}$ (model 1701). The Alza pumps are implanted subcutaneously in the loose fold of the neck of each test animal. The Alza pump contains a predetermined treatment dosage of the bovine somatomedin. Various dosages for each treatment are obtained by the appropriate dilution of the somatomedin preparation placed in the Alza pump. A more detailed description of the specific testing methods and the results therefrom are set out in the examples below.

In accordance with the practices of the present invention, the somatomedin of the invention may be administered alone or in admixture with various diluents, carriers, excipients or adjuvants suitably selected with respect to the intended route of administration and conventional practices. While the preferred method of administration is in a continuous manner as discussed above, the somatomedin of the present invention may be administered to the animals by other parenteral means.

The somatomedins of the present invention or preparations thereof may be administered to warm-blooded animals, i.e., economic animals, including cattle, sheep, hogs, rabbits, chickens and other like animals. Dosages sufficient to elicit the desired growth promoting effect and improved feed efficiency will generally range between about 0.01 microgram of bovine somatomedin per 1kg of body weight per day to about 50 micrograms of bovine somatomedin per 1kg of body weight per day. As previously indicated, it is preferred that the foregoing dosages be administered in a continuous manner.

Of course, it should be recognized that the actual effective dosage to be administered will vary, depending upon the specific age, weight and responsiveness of the particular animal species.

The following nonlimiting examples are afforded in order that those skilled in the art may more readily understand the present invention and specific preferred embodiments thereof with respect to the administration of bovine somatomedin (bSM) to economic animals in accordance with the foregoing description.

EXAMPLE 1

Bovine Somatomedin Administered by Subcutaneous Injection

For the purpose of this example, bovine somatomedin was administered twice daily by subcutaneous injection to young pituitary normal male mice. This example is also offered as a comparison with Example 2 where a continuous method of administration was utilized.

Each treatment consisted of a subcutaneous injection, administered in the loose fold of the neck (dorsal surface) by one cc tuberculin syringe and 25 gauge needle. Treatments were administered daily for seven days. Four classes of treatments were utilized with four replicates of five mice per treatment as follows:

    1)    vehicle only injected control (0.0 micrograms bSM).

    2)    1x treatment (5.0 micrograms bSM/1kg body weight/day).

    3)    1/4x treatment (1.25 micrograms bSM/1kg body weight/day).

    4)    1/16x treatment (0.31 microgram bSM/1kg body weight/day).

The treatment dosage was divided to provide half the treatment dose at 4 p.m. (16:00 hours) daily and the second half again at 8 a.m. (8:00 hours) daily. The first treatment was given at 4 p.m. on Day 1, the last treatment was given at 8 a.m. on Day 8. This regime required that 14 individual injections be given per mouse. The material administered was prepared in sterile physiological saline containing no more than $10^{-3}$M HCl.

This test compensated for growth dilution of the dose by providing treatment variation by volume of injection, based on average daily body weight.

The results are summarized in Table 1.

The "Body Weight Gained (g)" is the mean difference in grams (g) per replicate of 5 mice between the body weights on day 1 and on day 8.

The "Feed Consumed (g)" is the mean weight of feed (ground Purina$^{TM}$ mouse chow 9F) eaten per replicate of 5 mice during the treatment period.

The "Feed Efficiency (g/g)" is the mean of the ratio of feed consumed per body weight gained per replicate of 5 mice.


TABLE 1

Effect of Bovine Somatomedin, Injected S.C., on Body Weight
Gained, Feed Consumed, and Feed Efficiency of Male Mice


| TREATMENTS (micro-grams bSM/kg/day) | BODY WEIGHT GAINED (g) | FEED CONSUMED (g) | FEED EFFICIENCY (g/g) |
|---|---|---|---|
| 1) Vehicle Control | 40.3 | 147.2 | 3.69 |
| 2) 5.0 | 42.0 | 146.7 | 3.51 |
| 3) 1.25 | 38.7 | 147.3 | 3.84 |
| 4) 0.31 | 39.1 | 145.6 | 3.79 |


Improvements over these results are realized in the following example.


EXAMPLE 2

Bovine Somatomedin Administered by Subcutaneous Alza Pump Implant

In this example, bovine somatomedin was administered continuously by subcutaneous (S.C.) Alza pump implant to young pituitary normal rats.

Each treatment consisted of a single subcutaneous implant of an Alzet Minipump$^{TM}$ (prepared to contain bSM and/or vehicle as described), located in the loose fold of the neck under thiopenthal anesthesia. Four treatments of up to eight rats each were utilized:

    1)    Vehicle only implanted controls (0.0 micrograms bSM);

    2)    1x treatment (20.0 micrograms bSM/1kg body weight/day);

    3)    1/4x treatment (5.0 micrograms bSM/1kg body weight/day);

    4)    1/16x treatment (1.25 micrograms bSM/1kg body weight/day).

The following procedures were used:

    1)    Each rat was anesthetized by i.p. injections of thiopenthal (sodium pentothal) at 20mg/kg body weight.

2)    A one inch square section of hair was shaved (electric clipper) from the back (dorsal midline, thorax).

3)    A 1/2 to 3/4 inch cut (scissors) was made through the skin along the midline.

4)    The anterior subcutaneous space was opened.

5)    A filled Alza pump was inserted into this space.

6)    The wound was closed with one or two stainless wound clips as necessary.

7)    The rat was weighed, returned to its cage, and observed until recovery from anesthesia.

## Preparation of Treatments

All handling of bSM preparations were carried out in plastic ware to minimize losses due to surface adsorption of proteins. The material administered was prepared in sterile physiological saline solution containing no more than $10^{-3}$ M HCl.

The primary (1x) level treatment of bSM was formulated so that the initial rate of bSM delivery was set at 0.83 microgram bSM per kg body weight per hour. In other words, for a 50g rat containing 4.0ml serum, an Alza pump rate of one microliter per hour would provide a serum concentration of 10.4 nanogram bSM per milliliter of serum per hour. The lower concentration level treatments were prepared by dilution into sterile physiological saline solution.

On the morning of treatment day 0, each Alza pump with its flow moderator was tared empty, filled with the appropriate treatment solution, the flow moderator was inserted and the pump again weighed as an indication of the amount of treatment supplied by the pump. Each filled pump was immersed in sterile saline solution at room temperature until the animals were prepared for implantation.

The results are summarized in Tables 2 and 3.

TABLE 2

Effect of Bovine Somatomedin by S.C.
Alza Pump on Gain and Feed Efficiency in the Rat

TREATMENT (micrograms bSM/kg body weight/day)

| Parameter | 20.0 | 5.0 | 1.25 | 0.0 |
|---|---|---|---|---|
| Body Weight Gained (g) | 40.0 | 41.9 | 41.6 | 38.2 |
| Response, % | +4.7 | +9.7 | +8.9 | – |
| Feed Consumed (g) | 97.1 | 92.3 | 92.8 | 92.0 |
| Response, % | +5.6 | +0.4 | +0.9 | – |
| Feed/Gain (g/g) | 2.45 | 2.22 | 2.23 | 2.45 |
| Response, % | 0 | –9.4 | –9.0 | – |

TABLE 3

Effect of Bovine Somatomedin on
Terminal Percent Body Composition in the Rat

TREATMENT (micrograms bSM/kg body weight/day)

| Parameter | 20.0 | 5.0 | 1.25 | 0.0 |
|---|---|---|---|---|
| Moisture (%) | 74.67 | 74.84 | 75.22 | 75.23 |
| Protein (%) | 15.61 | 15.71 | 15.73 | 15.74 |
| Fat (%) | 5.06 | 4.91 | 4.56 | 4.61 |
| Ash (%) | 2.98 | 3.07 | 2.93 | 3.09 |

Table 2 provides the results of body weight gained, and feed efficiency per treatment. The values reported are averages from two trials. The results show that body weight gained is improved by bSM treatments. At 5.0 micrograms and 1.25 micrograms of bSM per kg per day of rat body weight, the gain response is significantly greater than the vehicle (0.0) control. Similarly, feed efficiency, as grams of feed consumed per gram of body weight gained is significantly improved at both the 5.0 microgram and 1.25 microgram bSM levels.

Table 3 shows the results of analyses of percents of moisture, protein, fat, and ash, as performed on the terminal whole body of each animal. The values reported, as in Table 2, are averages of treatment means from two trials. The results show that at 20.0 micrograms bSM per kg body weight per day the body percent of moisture is significantly decreased versus the vehicle control (0.0), while the percent of fat is significantly greater than control. Similarly, at 1.25 micrograms bSM, the ash content is significantly less than control.

While the invention has been described and illustrated with reference to certain preferred embodiments thereof, those skilled in the art will appreciate that various changes, modifications and substitutions can be made therein without departing from the spirit of the invention. For example, effective dosages other than the preferred ranges set forth hereinabove may be applicable as a consequence of variations and responsiveness of the animal treated, the age and weight of the animal, species specificity of the somatomedin, dosage related adverse affects, if any, observed and analogous considerations. It is intended, therefore, that the invention be limited only to the scope of the claims which follow.

CLAIMS:

1. A method for promoting growth and improving the feed efficiency of animals, characterized in that a growth-promoting effective amount of somatomedin is administered to a pituitary normal animal.

2. A method according to claim 1, wherein the administration is effected in a continuous manner.

3. A method according to claim 2, wherein the continuous administration is effected subcutaneously or intravenously.

4. A method according to claim 2 or 3, wherein the continuous administration is effected by means of an implanted minipump.

5. A method according to any preceding claim, wherein the amount of somatomedin administered is in the range from 50.0 to 0.01 microgram/1kg body weight/day.

6. A method according to any preceding claim, wherein the animals to which the somatomedin is administered are sheep, cattle, swine or chickens.

7. A preparation for promoting growth and improving the feed efficiency of animals, characterized by comprising a nontoxic carrier, diluent or adjuvant and somatomedin.

8. A preparation according to claim 7, wherein the somatomedin is a polypeptide having a molecular weight of about 8000 daltons and an isolectric point of about 8.5.

9. A preparation according to claim 7 or 8, wherein the carrier, diluent or adjuvant is a sterile physiological saline solution.

10. A preparation according to any of claims 7 to 9, which is in dosage unit form.

11. A preparation according to claim 10, wherein the dosage unit is arranged so as to provide, on administration, an amount of somatomedin in the range from 50.0 to 0.01 microgram/1kg body weight/day.

12. A preparation according to claim 11, wherein the dosage unit provides an amount of somatomedin in the range from 5.0 to 0.1 microgram/1kg body weight/day.